(19) Europäisches Patentamt
European Patent Office
Office européen des brevets

(11) Publication number: **0 152 131**
**B1**

(12) # EUROPEAN PATENT SPECIFICATION

(45) Date of publication of patent specification: **23.08.89**

(21) Application number: **85200067.8**

(22) Date of filing: **23.01.85**

(51) Int. Cl.$^4$: **C 07 D 213/82,**
C 07 D 241/24,
C 07 D 213/56, A 01 N 43/40,
A 01 N 43/60

(54) **Carboxamide derivatives, their preparation and their use as fungicides.**

(30) Priority: **13.02.84 GB 8403726**

(43) Date of publication of application:
**21.08.85 Bulletin 85/34**

(45) Publication of the grant of the patent:
**23.08.89 Bulletin 89/34**

(84) Designated Contracting States:
**AT BE CH DE FR GB IT LI LU NL SE**

(56) References cited:
**EP-A-0 059 536**
**EP-A-0 074 018**
**DE-A-2 519 532**
**DE-A-3 339 644**
**GB-A-2 011 414**
**US-A-3 991 071**

**Systemic Fungicides (Ed. R.W. MARSH) Longman, London, 1977, p.68-70**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Ten Haken, Pieter**
**Konkelboet The Street**
**Eastling Nr. Faversham Kent (GB)**
Inventor: **Pettman, Roger Bruce**
**5, Glovers Crescent**
**Sittingbourne Kent (GB)**

(74) Representative: **Hunter, Keith Roger Ian et al**
**4 York Road**
**London SE1 7NA (GB)**

(56) References cited:
**G.B. BARLIN, The Pyrazines, John Wiley, 1982, p. 8-10**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 152 131 B1

**Description**

This invention relates to carboxamide derivatives, to a process for their preparation, and to the use of such derivatives as fungicides.

With reference to earlier German Offenlegungsschrift No. 3339644, French Published Application No. 2535717, British Published Application No. 2129798 and Netherlands Published Application No. 8303808, the applicant has voluntarily limited the scope of the present application and submitted separate claims for the Federal Republic of Germany, France, United Kingdom and the Netherlands.

Certain carbamoyl compounds are known to exhibit fungicidal activity, and examples thereof are disclosed in UK Patent Application Publication No. 2,011,414A (carbamoyltriazoles) and in US Patents Nos. 3,991,071 and 4,080,462 (carbamoylimidazoles). One of these carbamoylimidazoles, 1-N-propyl-N-(2-(2,4,6-trichlorophenoxy)ethyl))carbamoylimidazole, is the protectant and eradicant fungicide prochloraz. There are also disclosed, in German Offenlegungsschrift No. 2519532, certain pyrazinylmethanol compounds which have fungicidal activity.

There has now been discovered a novel class of carboxamide derivatives having useful fungicidal activity.

According to the present invention there is provided a carboxamide derivative of general formula

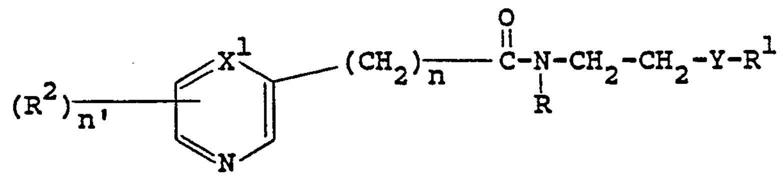

(I)

or a salt thereof wherein R represents a $C_{1-6}$alkyl, a $C_{3-8}$cycloalkyl or a benzyl group, Y is an oxygen atom, a sulphur atom, or a sulphinyl or sulphonyl group, $R^1$ is a phenyl group optionally substituted by a trifluoromethyl group or by 1 to 3 methyl groups or halogen atoms, n is 0 or 1, n' is 0 or 1, $R^2$ is a halogen moiety, and $X^1$ is a nitrogen atom or a carbon atom attached to a hydrogen atom, a halogen atom or a $C_{1-4}$alkoxy group.

$R^1$ is preferably a phenyl group optionally substituted by 1 to 3 methyl groups or halogen, e.g. bromine or chlorine, preferably chlorine, atoms.

Preferably n' is 0.

A preferred sub-group of derivatives of the invention comprises those wherein n' is 0, $R^1$ is a phenyl group optionally substitued by chlorine, bromine or methyl at at least one of the 2, 4 and 6-positions, R is a $C_{1-4}$alkyl group, a $C_{3-6}$cycloalkyl group or a benzyl group and $X^1$ is a nitrogen atom or a carbon atom attached to a hydrogen or chlorine atom or a methoxy group. A particularly advantageous sub-group comprises derivatives wherein $R^1$ is a phenyl group substituted by chlorine at at least one of the 2, 4 and 6-positions, n is 0, R is an ethyl or propyl group and $X^1$ is a carbon atom attached to a hydrogen atom or a methoxy group.

The salts of derivatives of formula I include metal salt complexes such as with $MnCl_2$.

Derivatives of formula I may be prepared by analogous processes to those known in the art for carboxamide derivatives.

The present invention further provides a process for the preparation of a derivative of formula I which comprises reacting an acid of formula

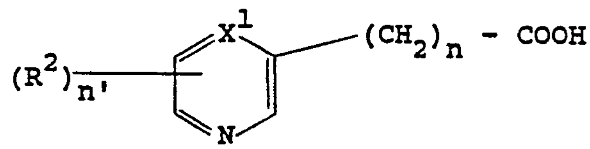

(II)

or a derivative thereof with an amide of formula

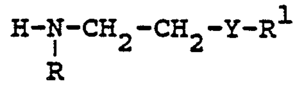

(III)

wherein R, Y, $R^1$, n, n', $R^2$ and $X^1$ are as defined above, in the presence of an inert solvent.

Acids of formula II and their derivatives are either known compounds, e.g. nicotinic acid, 3-pyridineacetic acid, or are obtainable by analogous processes to those for preparing known compounds.

Amines of formula III are similarly either known compounds or are obtainable by known processes, e.g. as described in US Patent No. 3,991,071.

Conveniently the derivative of the acid of formula II may be an acid halide, advantageously an acid chloride. When the acid of formula II is used *per se*, conveniently it is reacted with the amine of formula III in the presence of a carbodiimide.

Suitable inert solvents for the process include ethers, such as ether (diethyl ether), haloalkanes, such as methylene chloride and pyridine. Reaction may conveniently be effected at temperatures in the range from 0°C to ambient temperature.

Further in accordance with the invention there is provided a fungicidal composition which comprises a carrier and, as active ingredient, a derivative of formula I or a salt thereof as defined above.

A composition according to the invention preferably contains from 0.5 to 95% by weight of active ingredient.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating fungicidal compositions may be used.

Suitable solid carriers include natural silicas such as diatomaceous earths; magnesium silicates, for example talcs; magnesium aluminum silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Fungicidal compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75%w of active ingredient and usually contain, in addition to solid inert carrier, 3—10%w of a dispersing agent and, where necessary, 0—10%w of stabiliser(s) and/or other additives such as penetrants or stickers. Dusts are usually formulated as a dust concentrate having a similar composition to that of a wettable powder but without a dispersant, and may be diluted in the field with further solid carrier to give a composition usually containing ½—10%w of active ingredient. Granules are usually prepared to have a size between 10 and 100 BS mesh (1.676—0.152 mm), and may be manufactured by agglomeration or impregnation techniques.

Generally, granules will contain ½—25%w active ingredient and 0—10%w of additives such as stabilisers, slow release modifiers and binding agents. Emulsifiable concentrates usually contain, in addition to a solvent and, when necessary, co-solvent, 1—50%w/v active ingredient, 2—20%w/v emulsifiers and 0—20%w/v of other additives such as stabilisers, penetrants and corrosion inhibitors. Suspension concentrates are usually compounded so as to obtain a stable, non-sedimenting flowable product and usually contain 10—75%w of suspending agents such as protective colloids and thixotropic agents, 0—10%w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

The compositions may also contain other ingredients, for example other compounds possessing pesti-

cidal, especially insecticidal, acaricidal, herbicidal or fungicidal, properties.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The invention still further provides the use as a fungicide of a derivative of the general formula I or a salt or an ester thereof as defined above, and a method for combating fungus at a locus, which comprises treating the locus, which may for example be plants subject to or subjected to fungal attack, seeds of such plants or the medium in which such plants are growing or are to be grown, with such a derivative. Said plants are preferably wheat or barley.

The present invention is of wide applicability in the protection of crop plants against fungal attack. Typical crops which may be protected include vines, grain crops such as wheat and barley, and apples. The duration of protection is normally dependent on the individual compound selected, and also a variety of external factors, such as climate, whose impact is normally mitigated by the use of a suitable formulation.

The invention will be further understood from the following Examples.

## Example 1

N-nicotinyl-N-2-(2,4,6-trichlorophenoxyethyl)-N-propylamine

Nicotinic acid (3-pyridinecarboxylic acid) (1.0 g) and thionyl chloride (15 ml) were heated under reflux for 2 hours. Excess thionyl chloride was evaporated off under reduced pressure and the resulting residue was dissolved in dry ether (20 ml). The solution obtained was added dropwise at 0°C to a stirred solution of N-2-(2,4,6-trichlorophenoxyethyl)-N-propylamine (2.4 g) and triethylamine (0.9 g) in dry ether (30 ml). The reaction mixture was allowed to warm to ambient temperature (20°C) and was stirred for 2 hours. The reaction mixture was filtered and the filtrate was washed, first with a saturated sodium bicarbonate solution then with water, and was dried ($MgSO_4$). Volatiles were evaporated off and the residue was triturated with cold 40—60 petrol to yield the title product as a white solid (1.88 g, 60%).

## Examples 2 to 27

A number of additional compounds of the invention were prepared using processes similar to that of Example 1.

Structures and properties of the compounds of Examples 1 to 27 are given in Table I following.

Table 1

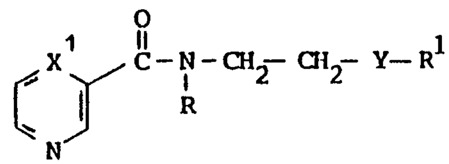

| Example | $R^1$ | Y | R | $X^1$ | mp (°C) | Yield (%) | Analysis Found (Theory) |
|---|---|---|---|---|---|---|---|
| 1 | 2,4,6-trichlorophenyl | O | *n*-propyl | CH | 102-104 | 60 | 52.7C, 4.4H, 7.2N (52.8C, 4.4H, 7.3N) |
| 2 | 2,4,6-trichlorophenyl | O | *i*-butyl | CH | oil | 36 | 53.1C, 4.7H, 7.0N (54.0C, 4.8H, 7.0N) |
| 3 | 2,4,6-trichlorophenyl | O | *n*-propyl | N | oil | 62 | 49.4C, 4.1H, 12.1N (49.0C, 4.4H, 11.8N) |
| 4 | 2,4,6-trichlorophenyl | O | ethyl | CH | 58-61 | 67 | 51.3C, 4.0H, 51.3C (51.4C, 4.0H, 7.5N) |
| 5 | 2,4,6-trichlorophenyl | O | methyl | CH | oil | 59 | 54.8C, 4.3H, 8.2N (55.7C, 4.0H, 8.7N) |

Table 1 (continued)

| Example | $R^1$ | Y | R | $X^1$ | mp (°C) | Yield (%) | Analysis Found (Theory) |
|---|---|---|---|---|---|---|---|
| 6 | 2,4,6-trichlorophenyl | O | *n*-propyl | C-Cl | 98-100 | 84 | 48.4C, 3.8H, 6.4N (48.3C, 3.8H, 6.6N) |
| 7 | 2,4,6-trichlorophenyl | O | *n*-propyl | $COCH_3$ | oil | 29 | 4.5H, 6.1N (4.5H, 6.7N) |
| 8 | 2,4-dichlorophenyl | O | *n*-propyl | CH | 75-77 | 65 | 57.9C, 5.1H, 8.0N (56.7C, 5.1H, 7.3N) |
| 9 | 2,4-dichlorophenyl | O | *i*-butyl | CH | 88-90 | 62 | 58.6C, 5.7H, 7.1N (59.0C, 5.5H, 7.6N) |
| 10 | 2,4-dichlorophenyl | O | *n*-propyl | N | oil | 58 | 54.3C, 4.9H, 11.7N (54.4C, 4.8H, 11.9N) |
| 11 | 2,4-dichlorophenyl | O | *i*-butyl | N | 48-50 | 69 | 55.1C, 5.3H, 11.4N (55.6C, 5.2H, 11.4N) |
| 12 | phenyl | S | *n*-propyl | CH | oil | 42 | 67.9C, 6.7H, 8.9N (68.0C, 6.7H, 9.3N) |

Table 1 (continued)

| Example | $R^1$ | Y | R | $X^1$ | mp (°C) | Yield (%) | Analysis Found (Theory) |
|---|---|---|---|---|---|---|---|
| 13 | 4-chlorophenyl | S | *n*-propyl | CH | oil | 66 | 61.5C, 5.7H, 8.0N (61.1C, 5.7H, 8.4N) |
| 14 | 4-chlorophenyl | SO | *n*-propyl | CH | oil | 91 | 58.1C, 5.1H, 8.2N (58.3C, 5.4H, 8.0N) |
| 15 | 4-chlorophenyl | $SO_2$ | *n*-propyl | CH | oil | 79 | 55.8C, 5.3H, 7.5N (55.7C, 5.2H, 7.7N) |
| 16 | 2,6-dimethylphenyl | 0 | ethyl | CH | 97-100 | 43 | 71.4C, 7.7H, 10.0N (72.5C, 7.4H, 9.4N) |
| 17 | 2,6-dimethylphenyl | 0 | *n*-propyl | CH | oil | 38 | 72.4C, 8.3H, 9.1N (73.1C, 7.7H, 9.0N) |
| 18 | 2,4,6-trichlorophenyl | 0 | cyclopropyl | CH | 99-102 | 39 | 52.6C, 3.9H, 7.2N (52.9C, 3.9H, 7.3N) |
| 19 | 2,4,6-tribromophenyl | 0 | ethyl | CH | 77-79 | 32 | 37.8C, 2.9H, 5.5N (37.9C, 3.0H, 5.5N) |

Table 1 (continued)

| Example | $R^1$ | Y | R | $X^1$ | mp (°C) | Yield (%) | Analysis Found (Theory) |
|---|---|---|---|---|---|---|---|
| 20 | 2,4,6-tribromophenyl | 0 | *n*-propyl | CH | 115-117 | 42 | 38.4C, 3.2H, 4.9N (39.1C, 3.3H, 5.4N) |
| 21 | 2,4,6-trichlorophenyl | 0 | *n*-butyl | CH | 115-117 | 25 | 53.5C, 4.7H, 6.7N (54.0C, 4.8H, 7.0N) |
| 22 | 2,4,6-trichlorophenyl | 0 | benzyl | CH | oil | 53 | 3.7H, 5.7N (3.9H, 6.5N) |
| 23 | 3-trifluoromethylphenyl | 0 | *n*-propyl | CH | oil | 24 | 61.2C, 5.7H, 7.7N (61.4C, 5.4H, 8.0N) |
| 24 | 2,6-dichlorophenyl | 0 | ethyl | CH | oil | 61 | 56.0C, 4.6H, 8.1N (56.6C, 4.7H, 8.3N) |
| 25 | 2,4,6-trichlorophenyl | 0 | cyclohexyl | CH | oil | 45 | 54.1C, 4.8H, 5.8N (56.3C, 4.9H, 6.6 N) |
| 26 | 2,4,6-trimethylphenyl | 0 | *n*-propyl | CH | oil | 63 | 72.4C, 8.3H, 8.5N (73.1C, 7.7H, 8.8N) |
| 27 | 2,4,6-trichlorophenyl | 0 | *n*-propyl $MnCl_2$ salt | CH | 130-133 | 73 | 3.6H, 5.6N (3.3H, 5.5N) |

Example 28

N-(3-pyridylacetyl)-N-2-(2,4,6-trichlorophenoxyethyl)-N-propylamine

3-Pyridylacetic acid (1.0 g) was stirred in dry methylene chloride (10 ml) containing N,N'-dicyclohexylcarbodiimide (2.0 g) and N-2-(2,4,6-trichlorophenoxyethyl)-N-propylamine' (1.2 g) for 12 hours at ambient temperature (20°C). Water (20 ml) was added, the reaction mixture was filtered and the organic layer was separated off, washed, first with a saturated sodium bicarbonate solution and then with water, and was dried ($MgSO_4$). Volatiles were evaporated off under reduced pressure and the residue was chromatographed on silica using ether as eluent to yield the title product as an oil (0.6 g, 20%).

| | | | | |
|---|---|---|---|---|
| *Analysis* | Found | 53.7C | 5.0H | 6.2N |
| | (Theory | 54.0C | 4.8H | 7.0N) |

Example 29

The fungicidal activity of compounds of the invention was investigated by means of the following tests.

(a) *Direct protectant activity against vine downy mildew (Plasmopara viticola; Pvp)*

The test is a direct protectant one, using a foliar spray. The lower surfaces of leaves of whole vine plants (cv Cabernet Sauvignon) are sprayed at a dosage of 1 kilogram of active material per hectare using a track sprayer which delivers 620 l/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

(b) *Direct protectant activity against vine grey mould (Botrytis cinerea; Bcp)*

The test is a direct protectant one using a foliar spray and is effected as described under (a), with the difference that the leaves are inoculated by spraying with an aqueous solution containing $10^5$ conidia/ml.

(c) *Activity against wheat leafspot (Leptosphaeria nodorum; Ln.)*

The test is a direct antisporulant one, using a foliar spray. Leaves of wheat plants (cv Mardler), at the single leaf stage, are inoculated by spraying with an aqueous suspension containing $8 \times 10^5$ spores/ml. The inoculated plants are kept for 24 hours in a high humidity compartment prior to treatment. The plants are sprayed at a dosage of 1 kg. of active material per hectare using a track sprayer as described under (a). After drying, the plants are kept for 5 days under normal glasshouse conditions, followed by assessment. Assessment is based on the percentage of leaf area covered by sporulation compared with that on leaves of control plants.

(d) *Activity against barley powdery mildew (Erysiphe graminis f.sp. hordei; Eg)*

The test is a direct antisporulant one, using a foliar spray. Leaves of barley seedlings, cultivar Golden Promise, are inoculated by dusting with mildew conidia one day prior to treatment with the test compound. The inoculated plants are kept overnight at glasshouse ambient temperature and humidity prior to treatment. The plants are sprayed at a dosage of 1 kg. of active material per hectare using a track sprayer as described under (a). After drying, plants are returned to a compartment at ambient temperature and humidity for up to 7 days, followed by assessment. Assessment is based on the percentage of leaf area covered by sporulation compared with that on leaves of control plants.

(e) *Activity against apple powdery mildew (Podosphaera leucotricha; Pl)*

The test is a direct anti-sporulant one using a foliar spray. The upper surfaces of leaves of whole apple seedlings are inoculated by spraying with an aqueous suspension containing $10^5$ conidia/ml 2 days prior to treatment with the test compound. The inoculated plants are immediately dried and kept at glasshouse ambient temperatures and humidity prior to treatment. The plants are sprayed at a dosage of 1 kilogram of active material per hectare using a track sprayer as described under (a). After drying the plants are returned to a compartment at ambient temperature and humidity for up to 9 days, followed by assessment. Assessment is based on the percentage of leaf area covered by sporulation compared with that on leaves of control plants.

(f) *Activity against seedling wheat blight (Fusarium culmorium; FcS)*

The test is an anti-sporulant one using a soil drench. Surface sterilised wheat seeds (var Waggoner) are inoculated by soaking in an aqueous suspension containing $7 \times 10^5$ spores/ml (60 g seed per 80 ml suspension) at 22°C for 6 hours. The seeds are then sown in pots (5 per pot) in sand at a depth of 1 cm. 1 day after inoculation and planting the active material is applied at a rate of 10 kg/ha by pouring on a soil drench (concentration 0.36 g/l active material in 12%v/v acetone/water) evenly over the sand. The pots are then transferred to glasshouse, kept at 25°C and watered sparingly. 21 days after inoculation the resulting seedlings are removed from the pots and their roots are gently washed. Visual assessment is made based on lesion development on stem base and upper roots in comparison with control seedlings.

The extent of disease control in all the above tests is expressed as a rating compared with a diluent sprayed control according to the criteria:—

0 = less than 50% disease control
1 = about 50—80% disease control
2 = greater than 80% disease control

Results of the above tests are given in Table II following:

Table II

| Compound Example | Fungicidal Activity | | | | | |
|---|---|---|---|---|---|---|
| | Pvp | Bcp | Ln | Eg | Pl | FcS |
| 1 | 1 | 1 | 2 | 2 | 2 | |
| 2 | | | 1 | | | |
| 3 | | | 1 | | | |
| 4 | 2 | 2 | 2 | 2 | 2 | |
| 5 | 1 | | | | 1 | |
| 6 | | | 1 | 1 | | |
| 7 | | | | 1 | 2 | |
| 8 | | | 1 | 2 | | |
| 9 | | | 1 | 1 | | |
| 10 | | | 1 | | | |
| 11 | 1 | | | | | |
| 12 | 1 | | | | | |
| 13 | | | | 1 | 2 | |
| 14 | | | | | 2 | |
| 15 | | | 1 | 1 | 2 | |
| 16 | | | | 2 | | 1 |
| 17 | | 2 | 1 | 2 | 1 | 2 |
| 18 | 1 | | 1 | 2 | 2 | |
| 19 | | | | 2 | | |
| 20 | | | 1 | 2 | 1 | |
| 21 | | 2 | 2 | 2 | | |
| 22 | | | 2 | 2 | 1 | |
| 23 | | | | | 1 | |
| 24 | | | 1 | 2 | | |
| 25 | | | | | 1 | |
| 26 | | | | | 2 | |
| 27 | | | 1 | 2 | | 1 |
| 28 | | | | | 1 | |

**Claims for the Contracting States: AT BE CH IT LI LU SE**

1. A carboxamide derivative of general formula

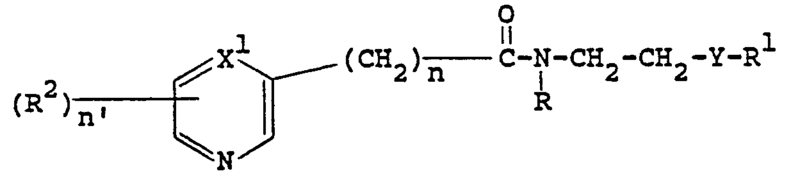

(I)

or a salt thereof, wherein R represents a $C_{1-6}$alkyl, a $C_{3-8}$cycloalkyl or a benzyl group, Y is an oxygen atom, a sulphur atom, or a sulphinyl or sulphonyl group, $R^1$ is a phenyl group optionally substituted by a trifluoromethyl group or by 1 to 3 methyl groups or halogen atoms, n is 0 or 1, n′ is 0 or 1, $R^2$ is a halogen moiety, and $X^1$ is a nitrogen atom or a carbon atom attached to a hydrogen atom, a halogen atom or a $C_{1-4}$alkoxy group.

2. A derivative according to Claim 1 wherein n′ is 0.

3. A derivative according to Claim 1 or Claim 2 wherein $R^1$ is a phenyl group optionally substituted by 1 to 3 halogen atoms or methyl groups.

4. A derivative according to Claim 3 wherein said halogen atoms are chlorine atoms.

5. A derivative according to any one of Claims 1 to 4 wherein n′ is 0, $R^1$ is a phenyl group optionally substituted by chlorine, bromine or methyl at at least one of the 2, 4 and 6-positions, R is a $C_{1-4}$alkyl group, a $C_{3-6}$cycloalkyl group or a benzyl group and $X^1$ is a nitrogen atom or a carbon atom attached to a hydrogen or chlorine atom or a methoxy group.

6. A derivative according to Claim 5 wherein $R^1$ is a phenyl group substituted by chlorine at at least one of the 2, 4 and 6-positions, n is 0, R is an ethyl or propyl group and $X^1$ is a carbon atom attached to a hydrogen atom or a methoxy group.

7. A process for the preparation of a derivative of formula I as defined in any one of Claims 1 to 6 which comprises reacting an acid of formula

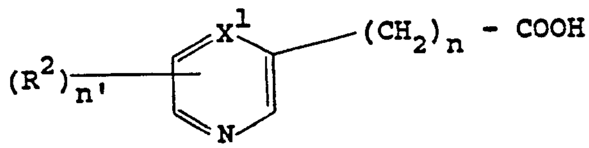

(II)

or a derivative thereof with an amine of formula

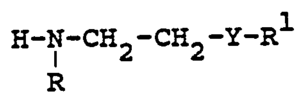

(III)

wherein R, Y, $R^1$, n, n′, $R^2$ and $X^1$ are as defined in Claim 1, in the presence of an inert solvent.

8. A process according to Claim 7 wherein the derivative of the acid of formula II is an acid halide.

9. A process according to Claim 7 wherein the acid of formula II is reacted with the amine of formula III in the presence of a carbodiimide.

10. A fungicidal composition which comprises a carrier and, as active ingredient, a derivative according to any one of Claims 1 to 6.

11. A method of combating fungus at a locus, which comprises treating the locus with a derivative of the general formula I or a salt thereof as defined in any one of Claims 1 to 6.

12. A method as claimed in Claim 11, in which the locus comprises plants subject or subjected to fungal attack, seeds of such plants or the medium in which the plants are growing or are to be grown.

13. A method as claimed in Claim 12 wherein said plants are wheat or barley.

14. The use as a fungicide of a derivative of the general formula I or a salt thereof as defined in any one of Claims 1 to 6.

**Claims for the Contracting States: DE FR GB NL**

1. A method of combating fungal infection in wheat or barley which comprises treating wheat or barley plants, seeds of such plants or the medium in which the plants are growing or are to be grown with a carboxamide derivative of general formula

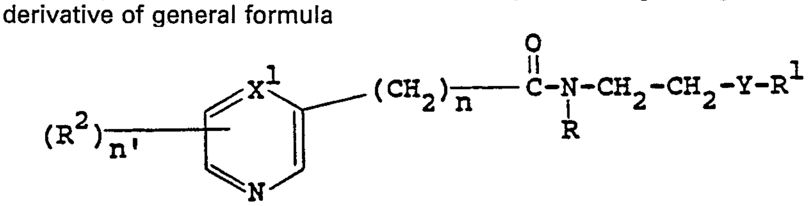

(I)

or a salt thereof, wherein R represents a $C_{1-6}$alkyl, a $C_{3-8}$cycloalkyl or a benzyl group, Y is an oxygen atom, a sulphur atom, or a sulphinyl or sulphonyl group, $R^1$ is a phenyl group optionally substituted by a trifluoromethyl group or by 1 to 3 methyl groups or halogen atoms, n is 0 or 1, n' is 0 or 1, $R^2$ is a halogen moiety, and $X^1$ is a nitrogen atom or a carbon atom attached to a hydrogen atom, a halogen atom or a $C_{1-4}$alkoxy group.

2. A method according to Claim 1 wherein n' is 0.

3. A method according to Claim 1 or Claim 2 wherein $R^1$ is a phenyl group optionally substituted by 1 to 3 halogen atoms or methyl groups.

4. A method according to Claim 3 wherein said halogen atoms are chlorine atoms.

5. A method according to any one of Claims 1 to 4 wherein n' is 0, $R^1$ is a phenyl group optionally substituted by chlorine, bromine or methyl at at least one of the 2, 4 and 6-positions, R is a $C_{1-4}$alkyl group, a $C_{3-6}$cycloalkyl group or a benzyl group and $X^1$ is a nitrogen atom or a carbon atom attached to a hydrogen or chlorine atom or a methoxy group.

6. A method according to Claim 5 wherein $R^1$ is a phenyl group substituted by chlorine at at least one of the 2, 4 and 6-positions, n is 0, R is an ethyl or propyl group and $X^1$ is a carbon atom attached to a hydrogen atom or a methoxy group.

7. A carboxamide derivative of the general formula I or a salt thereof, in which R, Y, $R^1$, n, n', $R^2$ and $X^1$ are as defined in Claim 1, with the provisos that, for compounds in which both n and n' are 0; when R is an n-propyl group, Y is an oxygen atom and $X^1$ is a nitrogen atom or a —CH— group then $R^1$ is not a 2,4,6-trichlorophenyl, 2,4-dichlorophenyl or 2,4,6-trimethylphenyl group; when R is an n-propyl group, $R^1$ is a 2,6-dichlorophenyl group and $X^1$ is a —CH— group then Y is not an oxygen or sulphur atom; and when R is an ethyl group, $R^1$ is a 2,4,6-trichlorophenyl group and Y is an oxygen atom then $X^1$ is not a nitrogen atom or a —CH— group.

8. A process for the preparation of a derivative of formula I as defined in Claim 7 which comprises reacting an acid of formula

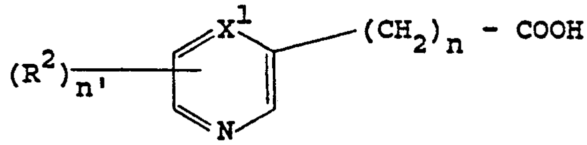

(II)

or a derivative thereof with an amine of formula

$$H-\underset{\substack{|\\R}}{N}-CH_2-CH_2-Y-R^1 \qquad (III)$$

wherein R, Y, $R^1$, n, n', $R^2$ and $X^1$ are as defined in Claim 1, in the presence of an inert solvent.

9. A process according to Claim 8, wherein the derivative of the acid of formula II is an acid halide.

10. A process according to Claim 8, wherein the acid of formula II is reacted with the amine of formula III in the presence of a carbodiimide.

11. A fungicidal composition which comprises a carrier and, as active ingredient, a derivative according to Claim 7.

12. A method of combating fungus at a locus, which comprises treating the locus with a derivative of the general formula I or a salt thereof as defined in Claim 7.

13. A method as claimed in Claim 12, in which the locus comprises plants subject or subjected to fungal attack, seeds of such plants or the medium in which the plants are growing or are to be grown.

14. The use as a fungicide of a derivative of the general formula I or a salt thereof as defined in Claim 7.

**Patentansprüche für die Vertragsstaaten: AT BE CH IT LI LU SE**

1. Carboxamid-Derivat der allgemeinen Formel

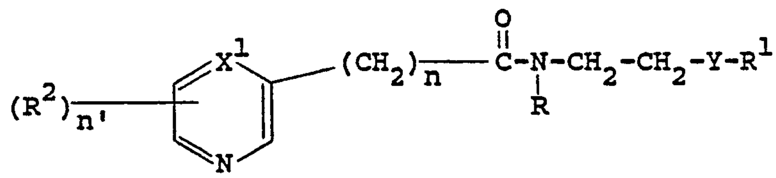

(I)

oder ein Salz hievon, worin R eine $C_{1-6}$Alkylgruppe eine $C_{3-8}$Cykloalkylgruppe oder eine Benzylgruppe bedeutet, Y ein Sauerstoffatom, ein Schwefelatom oder eine Sulfinyl- oder Sulfonylgruppe darstellt, $R^1$ eine Phenylgruppe bedeutet, die gegebenenfalls durch eine Trifluormethylgruppe oder durch 1 bis 3 Methylgruppen oder Halogenatome substituiert ist, n den Wert 0 oder 1 aufweist, n' den Wert 0 oder 1 hat, $R^2$ einen Halogenrest bedeutet und $X^1$ ein Stickstoffatom oder ein Kohlenstoffatom, das mit einem Wasserstoffatom, einem Halogenatom oder eine $C_{1-4}$Alkoxygruppe verbunden ist, darstellt.

2. Derivat nach Anspruch 1, worin n' den Wert 0 hat.

3. Derivat nach Anspruch 1 oder 2, worin $R^1$ eine Phenylgruppe bedeutet, die gegebenenfalls durch 1 bis 3 Halogenatome oder Methylgruppen substituiert ist.

4. Derivat nach Anspruch 3, worin die Halogenatome Chloratome sind.

5. Derivat nach einem der Ansprüche 1 bis 4, worin n' den Wert 0 hat, $R^1$ eine Phenylgruppe bedeutet, die gegebenenfalls durch Chlor, Brom oder Methyl an wenigstens einer der 2-, 4- und 6-Positionen substituiert ist, R eine $C_{1-4}$Alkylgruppe, eine $C_{3-6}$Cycloalkylgruppe oder eine Benzylgruppe darstellt und $X^1$ ein Stickstoffatom oder ein Kohlenstoffatom, das mit einem Wasserstoff- oder Chloratom oder mit einer Methoxygruppe verknüpft ist, bedeutet.

6. Derivat nach Anspruch 5, worin $R^1$ eine Phenylgruppe bedeutet, die an wenigstens einer der 2-, 4- und 6-Positionen durch Chlor substituiert ist, n den Wert 0 hat, R eine Ethyl- oder Propylgruppe ist und $X^1$ ein Kohlenstoffatom bedeutet, das mit einem Wasserstoffatom oder einer Methoxygruppe verknüpft ist.

7. Verfahren zur Herstellung eines Derivats der Formel I, wie in einem der Ansprüche 1 bis 6 definiert, welches Verfahren ein Umsetzen einer Säure der Formel

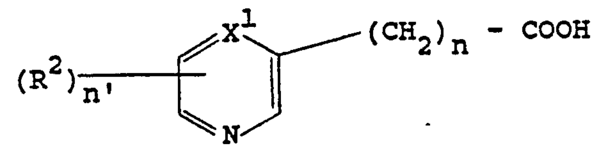

(II)

oder eines Derivates hievon mit einem Amin der Formel

$$H-\underset{\underset{R}{|}}{N}-CH_2-CH_2-Y-R^1 \qquad (III)$$

worin R, Y, $R^1$, n, n', $R^2$ und $X^1$ wie in Anspruch 1 definiert sind, in Gegenwart eines inerten Lösungsmittels umfaßt.

8. Verfahren nach Anspruch 7, worin das Derivat der Säure der Formel II ein Säurehalogenid ist.

9. Verfahren nach Anspruch 7, worin die Säure der Formel II mit dem Amin der Formel III in Gegenwart eines Carbodiimids umgesetzt wird.

10. Fungizide Zusammensetzung, welche einen Träger und, als wirksamen Bestandteil, ein Derivat nach einem der Ansprüche 1 bis 6 umfaßt.

11. Verfahren zur Bekämpfung von Fungus an einem Ort, welches ein Behandeln des Ortes mit einem Derivat der allgemeinen Formel I oder einem Salz hievon, wie in einem der Ansprüche 1 bis 6 definiert, umfaßt.

12. Verfahren nach Anspruch 11, worin der Ort Pflanzen, die einem Pilzbefall unterliegen oder unterliegen können, Samen solcher Pflanzen oder das Medium umfaßt, worin die Pflanzen wachsen oder gezogen werden sollen.

13. Verfahren nach Anspruch 12, worin die Pflanzen Weizen oder Gerste sind.

14. Verwendung eines Derivats der allgemeinen Formel I oder eines Salzes hievon, wie in einem der Ansprüche 1 bis 6 definiert, als ein Fungizid.

**Patentansprüche für die Vertragsstaaten: DE FR GB NL**

1. Verfahren zur Bekämpfung von Pilzbefall in Weizen oder Gerste, welches ein Behandeln von Weizen- oder Gerstenpflanzen, Samen solcher Pflanzen oder des Mediums, worin die Pflanzen wachsen oder gezogen werden sollen, mit einem Carboxamid-Derivat der allgemeinen Formel

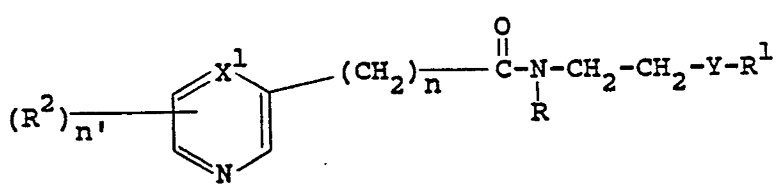

(I)

oder einem Salz hievon umfaßt, in welcher Formel R eine $C_{1-6}$Alkylgruppe, eine $C_{3-8}$Cykloalkylgruppe oder eine Benzylgruppe bedeutet, Y ein Sauerstoffatom, ein Schwefelatom oder eine Sulfinyl- oder Sulfonylgruppe darstellt, $R^1$ eine Phenylgruppe bedeutet, die gegebenenfalls durch eine Trifluormethylgruppe oder durch 1 bis 3 Methylgruppen oder Halogenatome substituiert ist, n den Wert 0 oder 1 aufweist, n' den Wert 0 oder 1 hat, $R^2$ einen Halogenrest bedeutet und $X^1$ ein Stickstoffatom oder ein Kohlenstoffatom, das mit einem Wasserstoffatom, einem Halogenatom oder eine $C_{1-4}$Alkoxygruppe verbunden ist, darstellt.

2. Verfahren nach Anspruch 1, worin n' den Wert 0 hat.

3. Verfahren nach Anspruch 1 oder 2, worin $R^1$ eine Phenylgruppe bedeutet, die gegebenenfalls durch 1 bis 3 Halogenatome oder Methylgruppen substituiert ist.

4. Verfahren nach Anspruch 3, worin die Halogenatome Chloratome sind.

5. Verfahren nach einem der Ansprüche 1 bis 4, worin n' den Wert 0 hat, $R^1$ eine Phenylgruppe bedeutet, die gegebenenfalls durch Chlor, Brom oder Methyl an wenigstens einer der 2-, 4- und 6-Positionen substituiert ist, R eine $C_{1-4}$Alkylgruppe, eine $C_{3-6}$Cycloalkylgruppe oder eine Benzylgruppe darstellt und $X^1$ ein Stickstoffatom oder ein Kohlenstoffatom, das mit einem Wasserstoff- oder Chloratom oder mit einer Methoxygruppe verknüpft ist, bedeutet.

6. Verfahren nach Anspruch 5, worin $R^1$ eine Phenylgruppe bedeutet, die an wenigstens einer der 2-, 4- und 6-Positionen durch Chlor substituiert ist, n den Wert 0 hat, R eine Ethyl- oder Propylgruppe ist und $X^1$ ein Kohlenstoffatom bedeutet, das mit einem Wasserstoffatom oder einer Methoxygruppe verknüpft ist.

7. Carboxamid-Derivat der allgemeinen Formel I oder ein Salz hievon, worin R, Y, $R^1$, n, n', $R^2$ und $X^1$ wie in Anspruch 1 definiert sind, mit der Maßgabe, daß für Verbindungen, worin sowohl n als auch n' den Wert 0 haben, gilt: wenn R eine n-Propylgruppe bedeutet, Y ein Sauerstoffatom ist und $X^1$ ein Stickstoffatom oder eine —CH-Gruppe darstellt, dann ist $R^1$ nicht eine 2,4,6-Trichlorphenylgruppe, 2,4-Dichlorphenylgruppe oder 2,4,6-Trimethylphenylgruppe; wenn R eine n-Propylgruppe darstellt, $R^1$ eine 2,6-Dichlorphenylgruppe ist und $X^1$ eine CH-Gruppe bedeutet, dann ist Y nicht ein Sauerstoff- oder Schwefelatom; und wenn R eine Ethylgruppe bedeutet, $R^1$ eine 2,4,6-Trichlorphenylgruppe darstellt und Y ein Sauerstoffatom bedeutet, dann ist $X^1$ nicht ein Stickstoffatom oder eine CH-Gruppe.

8. Verfahren zur Herstellung eines Derivats der Formel I, wie in Anspruch 7 definiert, welches Verfahren ein Umsetzen einer Säure der Formel

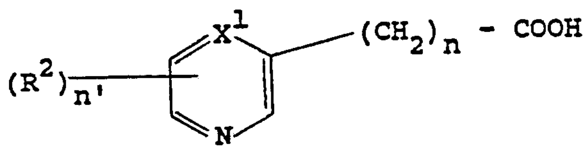

(II)

oder eines Derivates hievon mit einem Amin der Formel

$$H-\underset{R}{N}-CH_2-CH_2-Y-R^1 \qquad (III)$$

worin R, Y, $R^1$, n, n', $R^2$ und $X^1$ wie in Anspruch 1 definiert sind, in Gegenwart eines inerten Lösungsmittels umfaßt.

9. Verfahren nach Anspruch 8, worin das Derivat der Säure der Formel II ein Säurehalogenid ist.

10. Verfahren nach Anspruch 8, worin die Säure der Formel II mit dem Amin der Formel III in Gegenwart eines Carbodiimids umgesetzt wird.

11. Fungizide Zusammensetzung, welche einen Träger und, als wirksamen Bestandteil, ein Derivat nach Anspruch 7 umfaßt.

12. Verfahren zur Bekämpfung von Fungus an einem Ort, welches ein Behandeln des Ortes mit einem Derivat der allgemeinen Formel I oder einem Salz hievon, wie in Anspruch 7 definiert, umfaßt.

13. Verfahren nach Anspruch 12, worin der Ort Pflanzen, die einem Pilzbefall unterliegen oder unterliegen können, Samen solcher Pflanzen oder das Medium umfaßt, worin die Pflanzen wachsen oder gezogen werden sollen.

14. Verwendung eines Derivats der allgemeinen Formel I oder eines Salzes hievon, wie in Anspruch 7 definiert, als ein Fungizid.

**Revendications pour les Etats contractants: AT BE CH IT LI LU SE**

1. Un dérivé de carboxamide de la formule générale

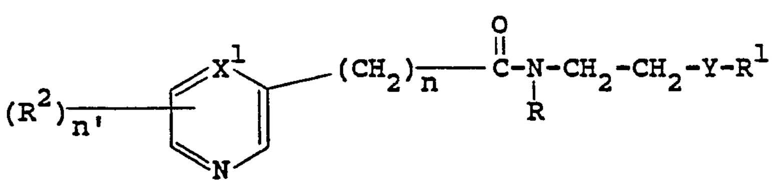

(I)

ou un de ses sels, dans laquelle R représente un groupe alkyle en $C_{1-6}$, une groupe cycloalkyle en $C_{3-8}$ ou un groupe benzyle, Y est un atome d'oxygène, un atome de soufre ou un groupe sulfinyle ou sulfonyle, $R^1$ est un groupe phényle optionnellement substitué par un groupe trifluorométhyl ou par 1 à 3 groupe(s) méthyle ou atome(s) d'halogène, n est 0 ou 1, n' est 0 ou 1, $R^2$ est une partie halogène, $X^1$ est un atome d'azote ou un atome de carbone lié à un atome d'hydrogène, un atome d'halogène ou un groupe alkoxy en $C_{1-4}$.

2. Un dérivé selon la revendication 1, dans lequel n' est 0.

3. Un dérivé selon la revendication 1 ou la revendication 2, dans lequel $R^1$ est un groupe phényle optionnellement substitué par 1 à 3 atomes d'halogène ou groupes méthyle.

4. Un dérivé selon la revendication 3, dans lequel lesdits atomes d'halogène sont des atomes de chlore.

5. Un dérivé selon l'une quelconque des revendications 1 à 4, dans lequel n' est 0, $R^1$ est un groupe phényle optionnellement substitué par un chlore, un brome ou un méthyle en au moins une des positions 2, 4 et 6, R est un groupe alkyle en $C_1$ à $C_4$, un groupe cycloalkyle en $C_3$ à $C_6$ ou un groupe benzyle et $X^1$ est un atome d'azote ou un atome de carbone lié à un atome d'hydrogène ou de chlore ou à un groupe méthoxy.

6. Un dérivé selon la revendication 5, dans lequel $R^1$ est un groupe phényle substitué par un chlore en au moins une des positions 2, 4 et 6, n est 0, R est un groupe éthyle ou propyle et $X^1$ est un atome de carbone lié à un atome d'hydrogène ou à un groupe méthoxy.

7. Un procédé pour la préparation d'un dérivé de formule I tel que défini dans l'une quelconque des revendications 1 à 6, qui consiste à faire réagir un acide de formule:

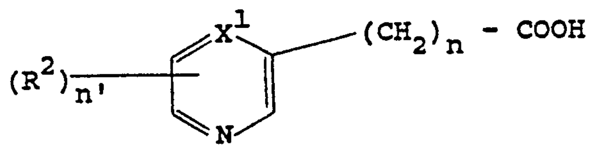

(II)

ou un de ses dérivés avec une amine de formule:

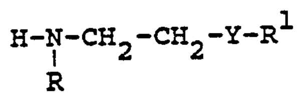

(III)

dans laquelle R, Y, $R^1$, n, n', $R^2$ et $X^1$ sont tels que définis dans la revendication 1, en présence d'un solvant inerte.

8. Un procédé selon la revendication 7, dans lequel le dérivé de l'acide de formule II est un halogénure d'acide.

9. Un procédé selon la revendication 7, dans lequel l'acide de formule II est mis à réagir avec l'amine de formule III en présence d'un carbodiimide.

10. Une composition fongicide qui comprend un support et, comme ingrédient actif, un dérivé selon l'une quelconque des revendications 1 à 6.

11. Une méthode pour combattre les champignons en un lieu, et qui consiste à traiter le lieu avec un dérivé de formule générale I ou un de ses sels tel que défini dans l'une quelconque des revendications 1 à 6.

12. Une méthode telle que revendiquée dans la revendication 11, dans laquelle le lieu comprend des plantes que l'on soumet ou qui sont soumises à une attaque fongique, des graines de ces plants ou le milieu dans lequel les plantes croissent ou doivent croître.

13. Une méthode telle que revendiqué dans la revendication 12, dans laquelle lesdites plantes sont le blé ou l'orge.

14. L'utilisation comme fongicide d'un dérivé de formule générale I ou d'un de ses sels tel que défini dans l'une quelconque des revendications 1 à 6.

**Revendications pour les Etats contractants: DE FR GB NL**

1. Une méthode pour combattre les infections fongiques du blé ou de l'orge que consiste à traiter les plantes de blé ou d'orge, les graines de ces plantes ou le milieu dans lequel les plantes croissent ou doivent croître avec un un dérivé de carboxamide de la formule générale

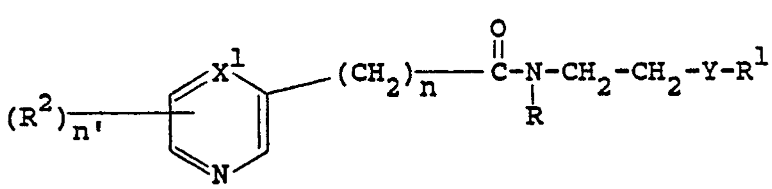

(I)

ou un de leurs sels, dans laquelle R représente un groupe alkyle en $C_{1-6}$, une groupe cycloalkyle en $C_{3-8}$ ou un groupe benzyle, Y est un atome d'oxygène, un atome de soufre ou un groupe sulfinyle ou sulfonyle, $R^1$ est un groupe phényle optionnellement substitué par un groupe trifluorométhyl ou par 1 à 3 groupes méthyle ou atomes d'halogène, n est 0 ou 1, n' est 0 ou 1, $R^2$ est une partie halogène, $X^1$ est un atome d'azote ou un atome de carbone lié à un atome d'hydrogène, un atome d'halogène ou un groupe alkoxy en $C_{1-4}$.

2. Une méthode selon la revendication 1, dans lequel n' est 0.

3. Un méthode selon la revendication 1 ou la revendication 2, dans lequel $R^1$ est un groupe phényle optionnellement substitué par 1 à 3 atomes d'halogène ou groupes méthyle.

4. Un méthode selon la revendication 3, dans lequel lesdits atomes d'halogène sont des atomes de chlore.

5. Un méthode selon l'une quelconque des revendications 1 à 4, dans lequel n' est 0, $R^1$ est un groupe phényle optionnellement substitué par du chlore, du brome ou du méthyle en au moins une des positions 2, 4 et 6, R est un groupe alkyle en $C_1$ à $C_4$, un groupe cycloalkyle en $C_3$ à $C_6$ ou un groupe benzyle et $X^1$ est un atome d'azote ou un atome de carbone lié à un atome d'hydrogène ou de chlore ou à un groupe méthoxy.

6. Un méthode selon la revendication 5, dans lequel $R^1$ est un groupe phényle substitué par du chlore en au moins une des positions 2, 4 et 6, n est 0, R est un groupe éthyle ou propyle et $X^1$ est un atome de carbone lié à un atome d'hydrogène ou à un groupe méthoxy.

7. Un dérivé de carboxamide de formule générale I ou un de ses sels, dans lequel R, Y, $R^1$, n, n', $R^2$ et $X^1$ sont tels que définis dans la revendication 1, à la condition que, pour des composés dans lesquels à la fois n et n' sont 0 lorsque R est un groupe n-propyle, Y est un atome d'oxygène et $X^1$ est un atome d'azote ou un groupe —CH— alors $R^1$ n'est pas un groupe 2,4,6-trichlorophényle, un groupe 2,4-dichlorophényle, ou un groupe 2,4,6-triméthylphényle; lorsque R est un groupe n-propyle, $R^1$ est un groupe 2,6-dichlorophényl et $X^1$ est est groupe —CH— alors Y est ni un atome d'oxygène ni un atome de soufre; et lorsque R est un groupe éthyle, $R^1$ est un groupe 2,4,6-trichlorophényl et Y est un atome d'oxygène alors $X^1$ n'est pas un atome d'oxygène ou un groupe —CH—.

8. Un procédé pour la préparation d'un dérivé de formule I tel que défini dans la revendication 7, qui consiste à faire réagir un acide de la formule:

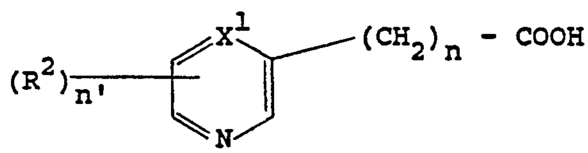

(II)

ou un de ses dérivés avec une amine de formule:

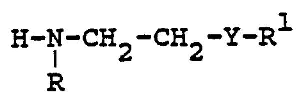

(III)

dans laquelle R, Y, $R^1$, n, n', $R^2$ et $X^1$ sont tels que définis dans la revendication 1, en présence d'un solvant inerte.

9. Un procédé selon la revendication 8, dans lequel le dérivé de l'acide de formule II est un halogénure d'acide.

10. Un procédé selon la revendication 8, dans lequel l'acide de formule II est mis à réagir avec l'amine de formule III en présence d'un carbodiimide.

11. Une composition fongicide qui comprend un support et, comme ingrédient actif, un dérivé selon la revendication 7.

12. Une méthode pour combattre les champignons en un lieu, et qui consiste à traiter le lieu avec un dérivé de la formule générale I ou un de ses sels tel que défini dans la revendication 7.

13. Une méthode telle que revendiquée dans la revendication 12, dans laquelle le lieu comprend des plantes que l'on soumet ou qui sont soumises à une attaque fongique, des graines de ces plants ou le milieu dans lequel les plantes croissent ou doivent croître.

14. L'utilisation comme fongicide d'un dérivé de formule générale I ou d'un de ses sels tel que défini dans la revendication 7.